# EUROPEAN PATENT APPLICATION

(11) **EP 1 312 379 A1**
(43) Date of publication of application: **21.05.2003**
(21) Application number: 01958449.9
(22) Date of filing: 24.08.2001
(51) Int. Cl.: A61K 45/00, A61K 31/4184, A61K 31/42, A61K 31/41, A61P 43/00, A61P 9/10, A61P 7/02

(54) **FIBRINOGEN LOWERING AGENTS**

(30) Priority: 25.08.2000 JP 2000260881
(71) Applicant: Takeda Chemical Industries, Ltd., Osaka-shi, Osaka 541-0045 (JP)
(72) Inventor: IMURA, Yoshimi, Toyono-gun, Osaka 563-0105 (JP); HIRAKATA, Masao, Kobe-shi, Hyogo 651-1143 (JP)
(74) Representative: Best, Michael, Dr.
(86) International application number: JP0107239
(87) International publication number: WO02015935

(57) **Abstract**

We offer a fibrinogen-lowering agent comprising a compound having an angiotensin II antagonistic activity, a prodrug thereof, or a salt thereof. Because of having an excellent effect of lowering fibrinogen, the above fibrinogen-lowering agent is useful as a prophylactic or therapeutic agent for various diseases caused by hyperfibrionogenemia, etc.

## Description

### Technical Field

The present invention relates to a fibrinogen-lowering agent and a prophylactic or therapeutic agent for hyperfibrinogenemia accompanied by hypercholesterolemia or hyperfibrinogenemia accompanied by a renal disorder comprising as an active component a compound having angiotensin II antagonistic activity (All antagonistic activity), a prodrug thereof, or a salt thereof.

### Background Art

Plasma fibrinogen (FIB) levels have been identified as an independent risk factor for cardiovascular diseases. It has been reported that some fibrates lower circulating plasma fibrinogen levels. Fibrates, such as fenofibrate, are, effective in lowering elevated plasma triglyceride levels. The action of fibrates on lipid metabolism is thought to be revealed via the activation of the peroxisome proliferator activated receptor-α (PPARα). Treatment of rats with fenofibrate decreased hepatic fibrinogen mRNA levels. In addition, plasma fibrinogen levels of PPARα-null mice were significant higher than those of wild-type mice. Thus, these results suggest that fibrate-suppressed plasma fibrinogen level is mediated through PPARα (Blood, 93(9), 2991-2998 (1999)).

All antagonistic activity are known to be therapeutic agents for circulatory system diseases such as hypertension, heart diseases (e.g. hypercardia, heart failure, and cardiac infarction), stroke, nephritis, etc (JP 4-364171 A, etc.). It is also known that a prolonged hypotensive effect can be obtained by blocking the action of All, which has a strong vasoconstrictive activity, on All receptor. However, no report suggests that compounds with All antagonistic activity have a fibrinogen-lowering activity.

### Disclosure of Invention

### (Object of the Invention)

There has been a demand- for a fibrinogen-lowering agent that has excellent medicinal properties such as, for example, excellent pharmacological effects for preventing and treating hyperfibrinogenemia and various diseases caused thereby without side effects.

### (Summary of the Invention)

Under the circumstances as described above, the present inventors have studied drugs effective in lowering fibrinogen intensively and, as a result, found that compounds having angiotensin II (AII) antagonistic activity, particularly those represented by a certain specific formula are highly effective in lowering fibrinogen.

The present inventors have also found that compounds having renin-angiotensin inhibitory activity including compounds having angiotensin II antagonistic activity can be effective in preventing or treating hyperfibrinogenemia accompanied by hypercholesterolemia or hyperfibrinogenemia accompanied by a renal disorder.

The present invention has been completed based on these findings.

That is, the present invention relates to:
(1) A fibrinogen lowering agent comprising a compound having angiotensin II antagonistic activity (exclusive of irbesartan), a prodrug thereof, or a salt thereof;
(2) The agent according to the above (1), wherein the compound having angiotensin II antagonistic activity is a non-peptide compound;
(3) The agent according to the above (1), wherein the compound having angiotensin II antagonistic activity is a compound having oxygen atom in its molecule;
(4) The agent according to the above (1), wherein the compound having angiotensin II antagonistic activity is a compound having ether linkage or carbonyl group in its molecule;
(5) The agent according to the above (1), wherein the compound having angiotensin II antagonistic activity is represented by the formula (I): 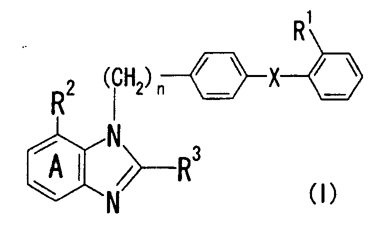 wherein R¹ is a group capable of forming an anion or a group convertible thereto, X shows that the phenylene group and the phenyl group are bonded directly or through a spacer having a chain length of 1 to 2 atoms, n is 1 or 2, ring A is benzene ring optionally further substituted, R² is a group capable of forming an anion or a group convertible thereto, and R³ is an optionally substituted hydrocarbon group which may be bonded through a hetero atom;
(6) The agent according to the above (1), wherein the compound having angiotensin II antagonistic activity is losartan, eprosartan, candesartan cilexetil, candesartan, valsartan, telmisartan, olmesartan, or tasosartan;
(7) The agent according to the above (1), wherein the compound having angiotensin II antagonistic activity is 2-etoxy-1-[[2'-(1H-tetrazol-5-yl)biphenyl-4-yl]methyl]benzimdazole-7-carboxylic acid;
(8) The agent according to the above (1), wherein the compound having angiotensin II antagonistic activity is 1-(cyclohexyloxycarbonyloxy)ethyl 2-ethoxy-1-[[2'-(1H-tetrazol-5-yl)biphenyl-4-yl]methyl]benzimidazole-7-carboxylate;
(9) The agent according to the above (1), wherein the compound having angiotensin II antagonistic activity is 2-ethoxy-1-[[2'-(2,5-dihydro-5-oxo-1,2,4-oxadiazol-3-yl)bipheny-4-yl]methyl]benzimidazole-7-carboxylic acid;
(10) The agent according to the above (1), which is a prophylactic or therapeutic agent for hyperfibrinogenemia or a disease caused thereby;
(11) A prophylactic or therapeutic agent for hyperfibrinogenemia accompanied by hypercholesterolemia, or hyperfibrinogenemia accompanied by a renal disorder, which comprises a compound having renin-angiotensin system inhibitory activity, a prodrug thereof, or a salt thereof;
(12) The agent according to the above (11), wherein the compound having renin-angiotensin system inhibitory activity is one or more compounds selected from the group consisting of (1) a compound having angiotensin II antagonistic activity, (2) a compound having angiotensin converting enzyme inhibitory activity, (3) a compound having renin inhibitory activity, (4) a compound having chymase inhibitory activity, and (5) a compound having aldosterone antagonistic activity;
(13) The agent according to the above (11), wherein the compound having renin-angiotensin system -inhibitory activity is a compound having angiotensin II antagonistic activity;
(14) The agent according to the above (13), wherein the compound having angiotensin II antagonistic activity is a non-peptide compound;
(15) The agent according to the above (13), wherein the compound having angiotensin II antagonistic activity is a compound having oxygen atom in its molecule;
(16) The agent according to the above (13), wherein the compound having angiotensin II antagonistic activity is a compound having ether linkage or carbonyl group in its molecule;
(17) The agent according to the above (13), wherein the compound having angiotensin II antagonistic activity is represented by the formula (I): 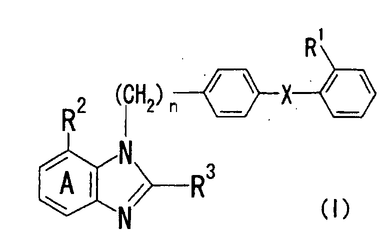 wherein R¹ is a group capable of forming an anion or a group convertible thereto, X shows that the phenylene group and the phenyl group are bonded directly or through a spacer having a chain length of 1 to 2 atoms, n is 1 or 2, ring A is benzene ring optionally further substituted, R² is a group capable of forming an anion or a group convertible thereto, and R³ is an optionally substituted hydrocarbon group which may be bonded through a hetero atom;
(18) The agent according to the above (13), wherein the compound having angiotensin II antagonistic activity is losartan, eprosartan, candesartan cilexetil, candesartan, valsartan, telmisartan, irbesaltan, olmesartan, or tasosartan;
(19) The agent according to the above (13), wherein the compound having angiotensin II antagonistic activity is 2-etoxy-1-[[2'-(1H-tetrazol-5-yl)biphenyl-4-yl]methyl]benzimdazole-7-carboxylic acid;
(20) The agent according to the above (13), wherein the compound having angiotensin II antagonistic activity is 1-(cyclohexyloxycarbonyloxy)ethyl 2-ethoxy-1-[[2'-(1H-tetrazol-5-yl)biphenyl-4-yl]methyl]benzimidazole-7-carboxylate;
(21) The agent according to the above (13), wherein the compound having angiotensin II antagonistic activity is 2-ethoxy-1-[[2'-(2,5-dihydro-5-oxo-1,2,4-oxadiazol-3-yl)bipheny-4-yl]methyl]benzimidazole-7-carboxylic acid;
(22) The agent according to the above (11), wherein the compound having renin-angiotensin system inhibitory activity is a compound having angiotensin converting enzyme inhibitory activity;
(23) The agent according to the above (22), wherein the compound having angiotensin converting enzyme inhibitory activity is enalapril, lisinopril, omapatrilat, sampatrilat, or adecut;
(24) The agent according to the above (22), wherein the compound having angiotensin converting enzyme inhibitory activity has both neutral endopeptidase inhibitory activity and angiotensin converting enzyme inhibitory activity;
(25) The agent according to the above (24), wherein the compound having both angiotensin converting enzyme inhibitory activity and neutral endopeptidase inhibitory activity is omapatrilat or sampatrilat;
(26) The agent according to the above (11), wherein the compound having renin-angiotensin system inhibitory activity is a compound having renin inhibitory activity;
(27) The agent according to the above (26), wherein the compound having renin inhibitory activity is SPP-100;
(28) The agent according to the above (11), wherein the compound having renin-angiotensin system inhibitory activity is a compound having chymase inhibitory activity;
(29) The agent according to the above (28), wherein the compound having chymase inhibitory activity is NK3201;
(30) The agent according to the above (11), wherein the compound having renin-angiotensin system inhibitory activity is a compound having aldosterone antagonistic activity;
(31) The agent according to the above (30), wherein the compound having aldosterone antagonistic activity is SC-66100;
(32) A method for preventing or treating hyperfibrinogenemia accompanied by hypercholesterolemia or hyperfibrinogenemia accompanied by a renal disorder, which comprises using a compound having renin-angiotensin system inhibitory activity, a prodrug thereof, or a salt thereof;
(33) Use of a compound having renin-angiotensin system inhibitory activity, a prodrug thereof, or a salt thereof, for manufacturing a prophylactic or therapeutic agent for hyperfibrinogenemia accompanied by hypercholesterolemia or hyperfibrinogenemia accompanied by a renal disorder;
(34) A method for lowering fibrinogen, which comprises using a compound having angiotensin II antagonistic activity (exclusive of irbesartan), a prodrug thereof, or a salt thereof;
(35) Use of a compound having angiotensin II antagonistic activity (exclusive of irbesartan), a prodrug thereof, or a salt thereof, for manufacturing a fibrinogen-lowering agent;
(36) A method for preventing or treating hyperfibrinogenemia or a disease caused thereby, which comprises administering an effective amount of a compound having angiotensin II antagonistic activity (exclusive of irbesartan), a prodrug thereof, or a salt thereof, to a mammal;
(37) Use of a compound having angiotensin II antagonistic activity (exclusive of irbesartan), a prodrug thereof, or a salt thereof, for manufacturing a prophylactic or therapeutic agent for hyperfibrinogenemia or a disease caused thereby; and the like.

### (Detailed Description of the Invention)

In the present invention, a compound having angiotensin II antagonistic activity, a prodrug thereof, or a salt thereof is advantageously used in lowering fibrinogen, etc.

In the present invention, angiotensin II antagonistic activity refers to the effects of competitively or non-competitively inhibiting the bonding of angiotensin II to an angiotensin II receptor on cell membranes to diminish potent vasoconstrictive activity or vascular smooth muscle growth activity induced by angiotensin II to alleviate hypertension symptoms.

The compound having angiotensin II antagonistic activity to be used for the present invention may be either a peptide compound or a non-peptide compound. In view of the advantage of long action, a non-peptide compound having angiotensin II antagonistic activity is preferable. As the compound having angiotensin II antagonistic activity, a compound having oxygen atom in its molecule is preferable, a compound having ether linkage or carbonyl group (said carbonyl group may form hydroxyl group by resonance) is more preferable, a compound having an ether linkage or a ketone derivative is further preferable, and in particular, an ether derivative is preferable.

Any non-peptide compound having angiotensin II antagonistic activity can be used for the present invention. Examples of said compounds include imidazole derivatives disclosed in JP 56-71073 A, JP 56-71074 A, JP 57-98270 A, JP 58-157768 A, USP 4,355,040, USP 4,340,598, etc.; modified imidazole derivatives disclosed in EP-253310, EP-291969, EP-324377, EP-403158, WO-9100277, JP 63-23868 A, JP 1-117876 A, etc.; pyrrole, pyrazole and triazole derivatives disclosed in USP 5,183,899, EP-323841, EP-409332, JP 1-287071 A, etc.; benzimidazole derivatives disclosed in USP 4,880,804, EP-0392317, EP-0399732, EP-0400835, EP-425921, EP-459136, JP 3-63264 A, etc.; azaindene derivatives disclosed in EP-399731, etc.; pyrimidone derivatives disclosed in EP-407342, etc.; quinazoline derivatives disclosed in E-P-411766, etc.; xanthine derivatives disclosed in EP-430300, etc.; fused imidazole derivatives disclosed in EP-434038, etc.; pyrimidinedione derivatives disclosed in EP-442473, etc.; thienopyridone derivatives disclosed in EP-443568, etc.; heterocyclic compounds disclosed in EP-445811, EP-483683, EP-518033, EP-520423, EP-588299, EP-603712, etc. In addition, their representative compounds are described in Journal of Medicinal Chemistry, Vol. 39, No. 3, pages 625-656 (1996). As the non-peptide compound having angiotensin II antagonistic activity, any one in addition to the compounds described in the above-mentioned references can be employed as far as it has angiotensin II antagonistic activity. Among others, Losartan (DuP753), Eprosartan (SK&F108566), Candesartan cilexetil (TCV-116), Valsartan (CGP-48933), Telmisartan (BIBR277), Irbesartan (SR47436), Tasosartan (ANA-756), Olmesartan (CS-866), their active metabolites (Candesartan, etc.), etc. are preferable.

Preferred examples of the non-peptide compound having angiotensin II antagonistic activity include, for example, a benzimidazole derivative of the formula (I): 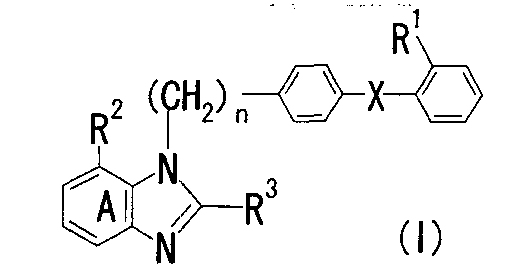 wherein R¹ is a group capable of forming an anion or a group capable of converting thereinto, X shows that the phenylene group and the phenyl group bind to each other directly or through a spacer having an atomic chain length of 2 or less, n is an integer of 1 or 2, ring A is benzene ring which may be further substituted, R² is a group capable of forming an anion or a group capable of converting thereinto, and R³ is an optionally substituted hydrocarbon residue which may bind through a hetero-atom (preferably, an optionally substituted hydrocarbon residue which binds through oxygen atom), etc., or a salt thereof.

In the above formula (I), the group capable of forming an anion (a group having hydrogen atom capable of leaving as a proton) as R¹ include, for example, (1) carboxyl group, (2) tetrazolyl group, (3) trifluoromethanesulfonic acid amido group (-NHSO₂CF₃), (4) phosphono group, (5) sulfo group, (6) an optionally substituted 5- to 7-membered (preferably 5- to 6-membered) monocyclic heterocyclic ring residue which contains one or more of N, S and O, etc.

Examples of the above "optionally substituted 5- to 7-membered (preferably 5- to 6-membered) monocyclic heterocyclic ring residue which contains one or more of N, S and 0" include 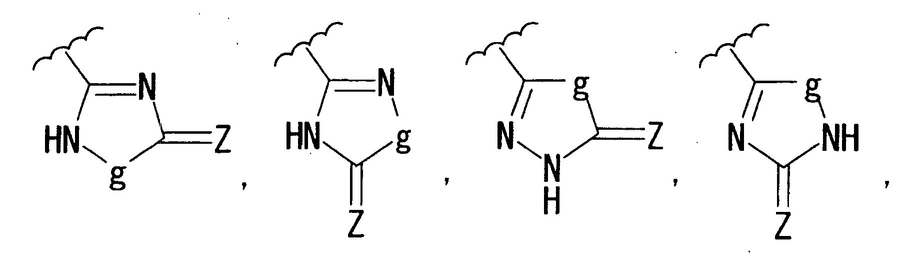 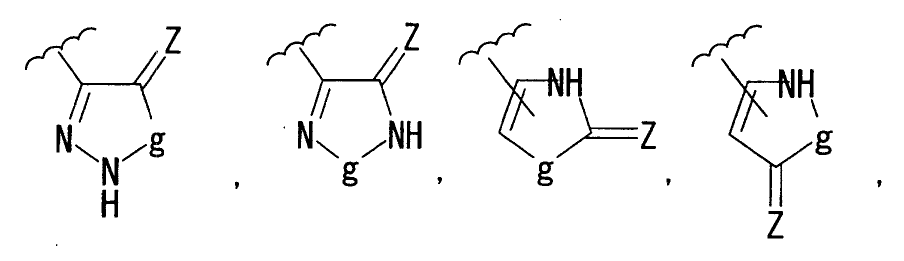 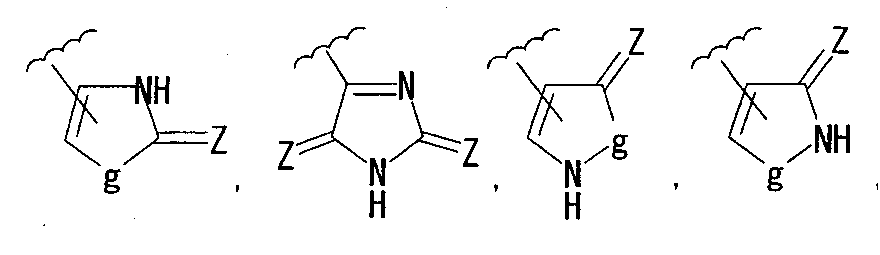 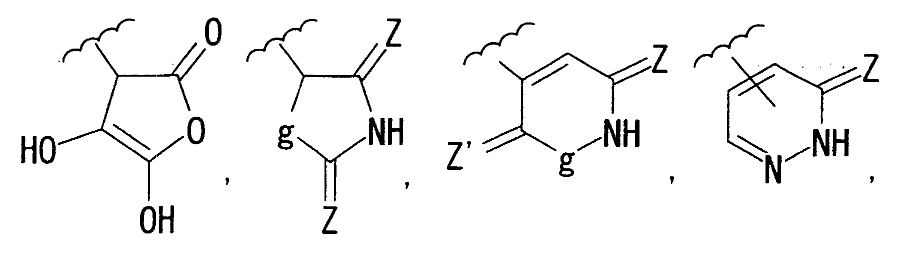 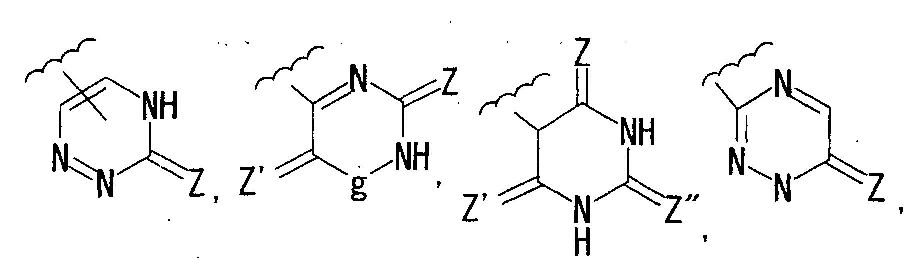 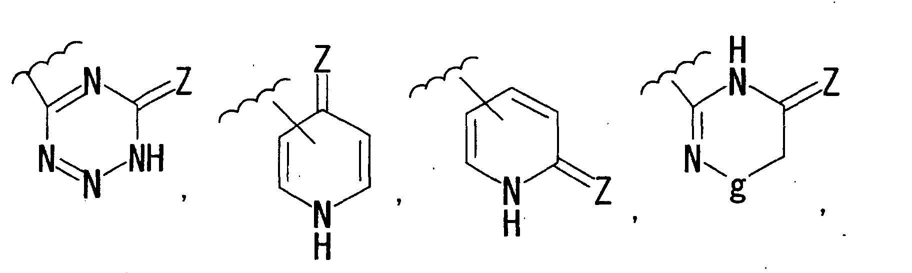 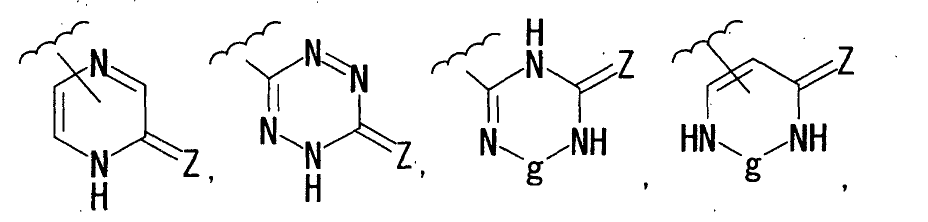 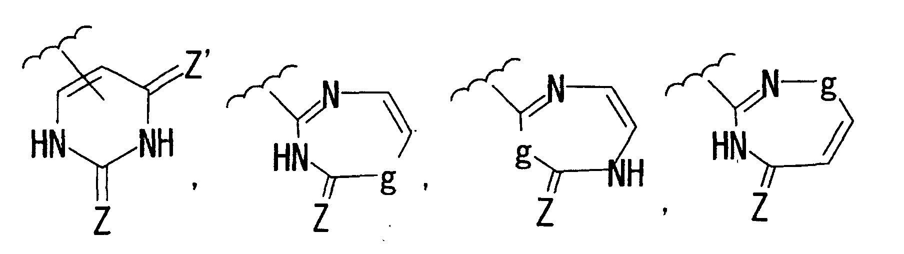 etc. The chemical bond between the heterocyclic ring residue represented by R¹ and the phenyl group to which said heterocyclic ring residue binds may be a carbon-carbon bond as shown above, or a nitrogen-carbon bond via one of the several nitrogen atoms when the symbol g is -NH-, etc. in the above formulas.

For example, when R¹ is represented by the formula: 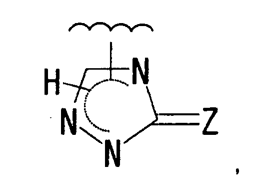 its specific embodiments are 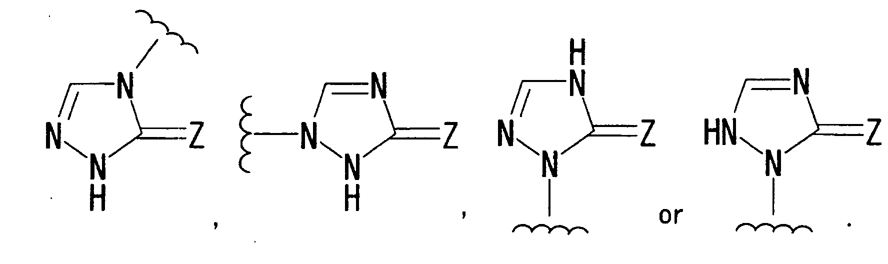 Other examples of R¹ binding through a nitrogen atom include 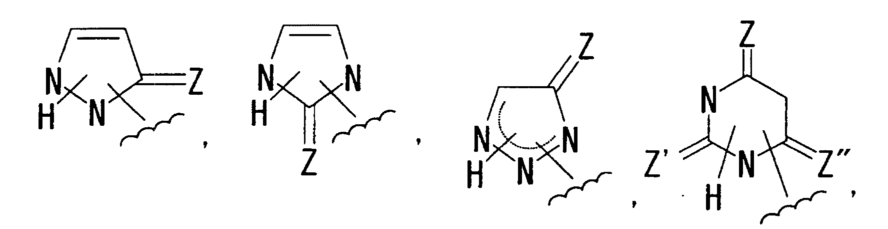 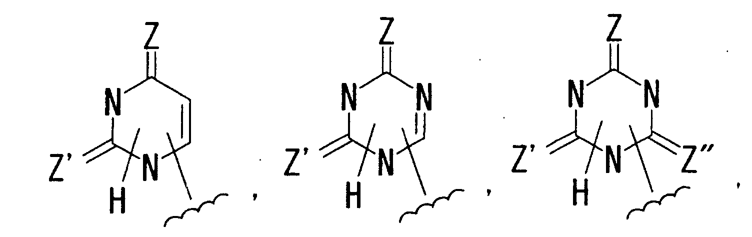 etc.

In the above formula, g is -CH₂-, -NH-, -O- or -S(O)m-; >=Z, >=Z' and >=Z'' are independently a carbonyl group, a thiocarbonyl group or an optionally oxidized sulfur atom (e.g., S, S(O), S(O)₂, etc.) (preferably a carbonyl group or a thiocarbonyl group, more preferably carbonyl group); and m is an integer of 0, 1 or 2.

Preferred examples of the heterocyclic ring residue represented by R¹ include a heterocyclic ring residue simultaneously having -NH- or -OH group as proton donor and carbonyl group, thiocarbonyl group, sulfinyl group, etc. as proton acceptor, such as oxadiazolone ring, oxadiazolothione ring or thiadiazolone ring, etc.

While the heterocyclic ring residue represented by R¹ may form a condensed ring by connecting the substituents on the heterocyclic ring, it is preferably 5- to 6-membered ring residue, more preferably 5-membered ring residue.

Preferred examples of the heterocyclic ring residue represented by R¹ include a group of the formula: 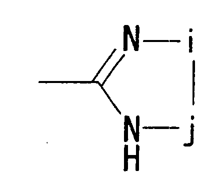 wherein i is -O- or -S-, j is >=O, >=S or >=S(O)m, and m is as defined above (preferably, 2,5-dihydro-5-oxo-1,2,4-oxadiazol--3-yl, 2,5-dihydro-5-thioxo-1,2,4-oxadiazol-3-yl, 2,5-dihydro-5-oxo-1,2,4-thiadiazol-3-yl; more preferably, 2,5-dihydro-5-oxo-1,2,4-oxadiazol-3-yl).

The above-mentioned heterocyclic ring residue (R¹) has the following tautomeric isomers. For example, in 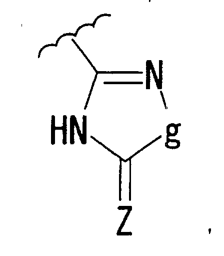 when Z is 0 and g is 0, 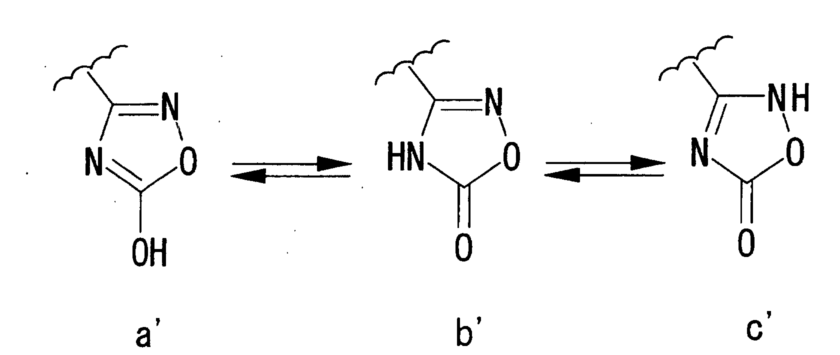 the three tautomeric isomers a', b' and c' exist and a group of the formula: 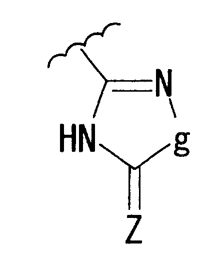 include all of the above a', b' and c'.

The group capable of forming an anion as R¹ may be protected by an optionally substituted lower (C₁₋₄) alkyl group, an acyl group (e.g., lower (C₂₋₅) alkanoyl, benzoyl, etc.), etc. at its possible position.

Examples of the optionally substituted lower (C₁₋₄) alkyl group include (1) a lower (C₁₋₄) alkyl group optionally substituted with one to three phenyl groups which may have halogen atom, nitro, lower (C₁₋₄) alkyl, lower (C₁₋₄) alkoxy, etc. (e.g., methyl, triphenylmethyl, p-methoxybenzyl, p-nitrobenzyl, etc.); (2) a lower (C₁₋₄) alkoxy-lower (C₁₋₄) alkyl group (e.g., methoxymethyl, ethoxymethyl, etc.); (3) a group of the formula: -CH(R⁴)-OCOR⁵ wherein R⁴ is (a) hydrogen, (b) a straight or branched lower C₁₋₆ alkyl group (e.g., methyl, ethyl, n-propyl, isopropyl, n-butyl, isobutyl, t-butyl, n-pentyl, isopentyl, neopentyl, etc.), (c) a straight or branched lower C₂₋₆ alkenyl group or (d) a C₃₋₈ cycloalkyl group (e.g., cyclopentyl, cyclohexyl, cycloheptyl, etc.), and R⁵ is (a) a straight or branched lower C₁₋₆ alkyl group (e.g., methyl, ethyl, n-propyl, isopropyl, n-butyl, isobutyl, sec-butyl, t-butyl, n-pentyl, isopentyl, neopentyl, etc.), (b) a straight or branched lower C₂₋₆ alkenyl group, (c) a lower C₁₋₃ alkyl group substituted with a C₃₋₈ cycloalkyl group (e.g., cyclopentyl, cyclohexyl, cycloheptyl, etc.) or an optionally substituted aryl group (e.g., a phenyl group, a naphthyl group, etc., optionally having a halogen atom, a nitro, a lower (C₁₋₄) alkyl, a lower (C₁₋₄) alkoxy, etc.) such as benzyl, p-chlorobenzyl, phenethyl, cyclopentylmethyl, cyclohexylmethyl, etc., (d) a lower C₂₋₃ alkenyl group substituted with a C₃₋₈ cycloalkyl or an optionally substituted aryl group (e.g., a phenyl group, a naphthyl group, etc., optionally having a halogen atom, a nitro, a lower (C₁₋₄) alkyl, a lower (C₁₋₄) alkoxy, etc.) such as cinnamyl, etc. having an alkenyl moiety such as vinyl, propenyl, allyl, isopropenyl, etc., (e) an optionally substituted aryl group (e.g., a phenyl group, naphthyl group, etc., optionally having a halogen atom, nitro, a lower (C₁₋₄) alkyl, a lower (C₁₋₄) alkoxy, etc.) such as phenyl, p-tolyl, naphthyl, etc., (f) a straight or branched lower C₁₋₆ alkoxy group (e.g., methoxy, ethoxy, n-propoxy, isopropoxy, n-butoxy, isobutoxy, sec-butoxy, t-butoxy, n-pentyloxy, isopentyloxy, neopentyloxy, etc.), (g) a straight or branched lower C₂₋₈ alkenyloxy group (e.g., allyloxy, isobutenyloxy, etc.), (h) a C₃₋₈ cycloalkyloxy group (e.g., cyclopentyloxy, cyclohexyloxy, cycloheptyloxy, etc.), (i) a lower C₁₋₃ alkoxy group substituted with a C₃₋₈ cycloalkyl (e.g., cyclopentyl, cyclohexyl, cycloheptyl, etc.) or an optionally substituted aryl group (e.g., phenyl group, naphthyl group, etc., optionally having a halogen atom, nitro, a lower (C₁₋₄) alkyl, a lower (C₁₋₄) alkoxy, etc.) such as benzyloxy, phenethyloxy, cyclopentylmethoxy, cyclohexylmethoxy, etc. having an alkoxy moiety such as methoxy, ethoxy, n-propoxy, isopropoxy, etc., etc.), (j) a lower C₂₋₃ alkenyloxy group substituted with a C₃₋₈ cycloalkyl (e.g., cyclopentyl, cyclohexyl, cycloheptyl, etc.) or an optionally substituted aryl group (e.g., phenyl group or naphthyl group, etc., optionally having a halogen atom, a nitro, a lower (C₁₋₄) alkyl, a lower (C₁₋₄) alkoxy, etc.) such as cinnamyloxy, etc. having an alkenyloxy moiety such as vinyloxy, propenyloxy, allyloxy, isopropenyloxy, etc. or (k) an optionally substituted aryloxy group (e.g., a phenoxy group, a naphthoxy group, etc., optionally having a halogen atom, nitro, a lower (C₁₋₄) alkyl, a lower (C₁₋₄) alkoxy, etc.) such as phenoxy, p-nitrophenoxy, naphthoxy, etc.; etc.

The group capable of forming an anion as R¹ may be substituted, in addition to the above protective group such as an optionally substituted lower (C₁₋₄) alkyl group or an acyl group (e.g., lower (C₂₋₅) alkanoyl, benzoyl, etc.), etc., with an optionally substituted lower (C₁₋₄) alkyl group (e.g. an optionally substituted lower (C₁₋₄) alkyl group similar to the "optionally substituted lower (C₁₋₄) alkyl group" exemplified as a protective group for the above group capable of forming an anion as R¹), a halogen atom, nitro, cyano, a lower (C₁₋₄) alkoxy, an amino optionally substituted with 1 to 2 lower (C₁₋₄) alkyl groups, etc., at the possible position.

In the above formula, the group convertible into the group capable of forming an anion (a group having a hydrogen atom capable of leaving as proton) as R¹ may be a group convertible into a group capable of forming an anion under biological or physiological conditions (for example, in vivo reaction, etc. such as oxidation, reduction, hydrolysis, etc. by in vivo enzyme, etc.) [so called pro-drug], or the group convertible into a group capable of forming an anion represented by R¹ may be a group chemically convertible into a group capable of forming an anion, such as cyano, N-hydroxycarbamimidoyl group (-C(=N-OH)-NH₂), a group selected from the class consisting of (1) carboxyl group, (2) tetrazolyl group, (3) trifluoromethanesulfonic acid amido group (-NHSO₂CF₃), (4) phosphono group, (5) sulfo group and (6) an optionally substituted monocyclic 5- to 7-membered (preferably 5- to 6-membered) monocyclic heterocyclic ring residue which contains one or more of N, S and O, each of which is protected with an optionally substituted lower (C₁₋₄) alkyl group or an acyl group, etc. [so called synthetic intermediate].

As the group R¹, carboxyl, tetrazolyl or 4,5-dihydro-5-oxo-1,2,4-oxadiazol-3-yl (preferably, tetrazolyl), each of which may be protected with an optionally substituted lower (C₁₋₄) alkyl (e.g., methyl, triphenylmethyl, methoxymethyl, ethoxymethyl, p-methoxybenzyl, p-nitrobenzyl, etc.) or an acyl group (e.g., lower (C₂₋₅) alkanoyl, benzoyl, etc.); or cyano or N-hydroxycarbamimidoyl (preferably cyano) is preferable. Among others, cyano is preferable.

In the above formula, X shows that the phenylene group is bonded to the adjacent phenyl group directly or through a spacer with an atomic chain of 2 or less (preferably directly). Examples of the spacer with an atomic chain of 2 or less include any divalent chain in which the number of atoms constituting the straight chain is 1 or 2 and which may have a side chain, and specifically lower (C₁₋₄) alkylene in which the number of atoms constituting the straight chain is 1 or 2, -CO-, -O-, -S-, -NH-, -CO-NH- , -O-CH₂-, -S-CH₂-, -CH=CH-, etc.

In the above formula, n is an integer of 1 or 2 (preferably 1).

In the above formula, ring A may have, in addition to the group R², another substituent, for example, (1) halogen (e.g., F, Cl, Br, etc.), (2) cyano, (3) nitro, (4) an optionally substituted lower (C₁₋₄) alkyl, (5) a lower (C₁₋₄) alkoxy, (6) an optionally substituted amino group (e.g., amino, N-lower (C₁₋₄) alkylamino (e.g., methylamino, etc.), N,N-di-lower (C₁₋₄) alkylamino (e.g., dimethylamino, etc.), N-arylamino (e.g., phenylamino, etc.), alicyclic amino (e.g., morpholino, piperidino, piperazino, N -phenylpiperazino, etc.), etc.), (7) a group of the formula: -CO-D' wherein D' is a hydroxyl group or a lower (C₁₋₄) alkoxy whose alkyl moiety may be substituted with a hydroxyl group, a lower (C₁₋₄) alkoxy, a lower (C₂₋₆) alkanoyloxy (e.g., acetoxy, pivaloyloxy, etc.), a lower (C₁₋₆) alkoxy-carbonyloxy (e.g., methoxycarbonyloxy, ethoxycarbonyloxy, etc.) or a lower (C₃₋₆) cycloalkoxycarbonyloxy (e.g., cyclohexyloxycarbonyloxy, etc.), or (8) tetrazolyl, trifluoromethanesulfonic acid amide group, phosphono group or sulfo group, each of which may be protected with an optionally substituted lower (C₁₋₄) alkyl ("an optionally substituted lower (C₁₋₄) alkyl group" similar to that exemplified as a protective group for the above group capable of forming an anion represented by R¹, etc.) or acyl (e.g., lower (C₂₋₅) alkanoyl, benzoyl, etc.), etc.

Of these substituents, one or two may simultaneously be present at any possible position on the benzene ring, and preferred examples of the substituents for the benzene ring represented by A in addition to the group R² include an optionally substituted lower (C₁₋₄) alkyl (e.g., a lower (C₁₋₄) alkyl, etc. optionally substituted with a hydroxyl group, a carboxyl group, a halogen, etc.), a halogen , etc. As ring A, benzene ring having no substituent in addition to the group R² is preferable.

In the above formula, examples of the group capable of forming an anion (a group having a hydrogen atom capable of leaving as proton) as R² include (1) an optionally esterified or amidated carboxyl group, (2) tetrazolyl group, (3) trifluoromethanesulfonic acid amido group (-NHSO₂CF₃), (4) phosphono group, (5) sulfo group, etc., each of which may be protected with an optionally substituted lower alkyl group (e.g. an optionally substituted lower (C₁₋₄) alkyl group similar to the "optionally substituted lower (C₁₋₄) alkyl group" exemplified as a protective group for the above group capable of forming an anion as R¹) or an acyl group (e.g., lower (C₂₋₅) alkanoyl, benzoyl, etc.), or any one of the groups capable of converting thereinto under biological or physiological conditions (for example, in vivo reaction, etc. such as oxidation, reduction, hydrolysis, etc. by in vivo enzyme, etc.), or chemically.

Examples of the optionally esterified or amidated carboxyl as R² include a group of the formula: -CO-D wherein D is (1) a hydroxyl group, (2) an optionally substituted amino (for example, amino, N-lower (C₁₋₄) alkylamino, N,N-di-lower (C₁₋₄) alkylamino, etc.) or (3) an optionally substituted alkoxy [e.g., (i) a lower (C₁₋₆) alkoxy group whose alkyl moiety is optionally substituted with hydroxyl group, an optionally substituted amino (e.g., amino, N-lower (C₁₋₄) alkylamino, N,N-di-lower (C₁₋₄) alkylamino, piperidino, morpholino, etc.), a halogen, a lower (C₁₋₆) alkoxy, a lower (C₁₋₆) alkylthio, a lower (C₃₋₈) cycloalkoxy or an optionally substituted dioxolenyl (e.g., 5-methyl-2-oxo-1,3-dioxolen-4-yl, etc.), or (ii) a group of the formula: -O-CH(R⁶)-OCOR⁷ wherein R⁶ is (a) hydrogen, (b) a straight or branched C₁₋₆ lower alkyl group (e.g., methyl, ethyl, n-propyl, isopropyl, n-butyl, isobutyl, t-butyl, n-pentyl, isopentyl, neopentyl, etc.), (c) a straight or branched C₂₋₆ lower alkenyl group or (d) a C₃₋₈ cycloalkyl group (e.g., cyclopentyl, cyclohexyl, cycloheptyl, etc.), and R⁷ is (a) a straight or branched C₁₋₆ lower alkyl group (e.g., methyl, ethyl, n-propyl, isopropyl, n-butyl, isobutyl, sec-butyl, t-butyl, n-pentyl, isopentyl, neopentyl, etc.), (b) a straight or branched C₂₋₆ lower alkenyl group, (c) a lower C₁₋₃ alkyl group substituted with a C₃₋₈ cycloalkyl group (e.g., cyclopentyl, cyclohexyl, cycloheptyl, etc.) or an optionally substituted aryl group (e.g., phenyl group, naphthyl group, etc., optionally having a halogen atom, a nitro, a lower (C₁₋₄) alkyl, a lower (C₁₋₄) alkoxy, etc.) such as benzyl, p-chlorobenzyl, phenethyl, cyclopentylmethyl, cyclohexylmethyl, etc., (d) a lower C₂₋₃ alkenyl group substituted with a C₃₋₈ cycloalkyl or an optionally substituted aryl group (e.g., phenyl group, naphthyl group, etc., optionally having a halogen atom, a nitro, a lower (C₁₋₄) alkyl, a lower (C₁₋₄) alkoxy, etc.) such as cinnamyl, etc. having an alkenyl moiety such as vinyl, propenyl, allyl, isopropenyl, etc., (e) an optionally substituted aryl group (e.g., phenyl group, naphthyl group, etc., optionally having a halogen atom, nitro, a lower (C₁₋₄) alkyl, a lower (C₁₋₄) alkoxy, etc.) such as phenyl, p-tolyl, naphthyl, etc., (f) a straight or branched lower C₁₋₆ alkoxy group (e.g., methoxy, ethoxy, n-propoxy, isopropoxy, n-butoxy, isobutoxy, sec-butoxy, t-butoxy, n-pentyloxy, isopentyloxy, neopentyloxy, etc.), (g) a straight or branched lower C₂₋₈ alkenyloxy group (e.g., allyloxy, isobutenyloxy, etc.), (h) a C₃₋₈ cycloalkyloxy group (e.g., cyclopentyloxy, cyclohexyloxy, cycloheptyloxy, etc.), (i) a lower C₁₋₃ alkoxy group substituted with a C₃₋₈ cycloalkyl (e.g., cyclopentyl, cyclohexyl, cycloheptyl, etc.) or an optionally substituted aryl group (e.g., phenyl group, naphthyl group, etc., optionally having a halogen atom, nitro, a lower (C₁₋₄) alkyl, a lower (C₁₋₄) alkoxy, etc.) such as benzyloxy, phenethyloxy, cyclopentylmethoxy, cyclohexylmethoxy, etc. having an alkoxy moiety such as methoxy, ethoxy, n-propoxy, isopropoxy, etc., etc.), (j) a lower C₂₋₃ alkenyloxy group substituted with a C₃₋₈ cycloalkyl (e.g., cyclopentyl, cyclohexyl, cycloheptyl, etc.) or an optionally substituted aryl group (e.g., phenyl group or naphthyl group, etc., optionally having a halogen atom, a nitro, a lower (C₁₋₄) alkyl, a lower (C₁₋₄) alkoxy, etc.) such as cinnamyloxy, etc. having an alkenyloxy moiety such as vinyloxy, propenyloxy, allyloxy, isopropenyloxy, etc. or (k) an optionally substituted aryloxy group (e.g., a phenoxy group, a naphthoxy group, etc., optionally having a halogen atom, a nitro, a lower (C₁₋₄) alkyl, a lower (C₁₋₄) alkoxy, etc.) such as phenoxy, p-nitrophenoxy, naphthoxy, etc.], etc.

As R², an optionally esterified carboxyl is preferable, and its specific examples include -COOH and a salt thereof, -COOMe, -COOEt, -COOtBu, -COOPr, pivaloyloxymethoxycarbonyl, 1-(cyclohexyloxycarbonyloxy)ethoxycarbonyl, 5-methyl-2-oxo-1,3-dioxolen-4-ylmethoxycarbonyl, acetoxymethoxycarbonyl, propionyloxymethoxycarbonyl, n-butyryloxymethoxycarbonyl, isobutyryloxymethoxycarbonyl, 1-(ethoxycarbonyloxy)ethoxycarbonyl, 1-(acetoxy)ethoxycarbonyl, 1-(isobutyryloxy)ethoxycarbonyl, cyclohexylcarbonyloxymethoxycarbonyl, benzoyloxymethoxycarbonyl, cinnamyloxycarbonyl, cyclopentylcarbonyloxymethoxycarbonyl, etc. The group R² may be any one of the groups capable of forming an anion under biological or physiological conditions (for example, in vivo reaction, etc. such as oxidation, reduction, hydrolysis, etc. by in vivo enzyme, etc.), the groups capable of chemically forming an anion (e.g., COO-, its derivative, etc.) or the groups capable of converting thereinto. The group R² may be a carboxyl group or its pro-drug.

Preferred examples of the group R² include a group of the formula: -CO-D wherein D is (1) hydroxyl group or (2) a lower (C₁₋₄) alkoxy whose alkyl moiety is optionally substituted with a hydroxyl group, an amino, a halogen , a lower (C₂₋₆) alkanoyloxy (e.g., acetoxy, pivaloyloxy , etc.), a lower (C₃₋₈) cycloalkanoyloxy, a lower (C₁₋₆) alkoxycarbonyloxy (e.g., methoxycarbonyloxy, ethoxycarbonyloxy, etc.), a lower (C₃₋₈) cycloalkoxycarbonyloxy (e.g., cyclohexyloxycarbonyloxy, etc.), a lower (C₁₋₄) alkoxy or a lower (C₃₋₈) cycloalkoxy. Among others, an esterified carboxyl with a lower (C₁₋₄) alkyl (preferably, methyl or ethyl) is preferable.

In the above formula, examples of the "hydrocarbon residue" in the "optionally substituted hydrocarbon residue which may bind through a hetero-atom" represented by R³ include (1) an alkyl group, (2) an alkenyl group, (3) an alkynyl group, (4) a cycloalkyl group, (5) an aryl group, (6) an aralkyl group, etc. Among others, an alkyl group, an alkenyl group and a cycloalkyl group are preferable.

Examples of the alkyl group of the above mentioned (1) include straight or branched lower alkyl group having about 1-8 carbon atoms such as methyl, ethyl, propyl, isopropyl, butyl, isobutyl, sec-butyl, t-butyl, pentyl, i-pentyl, hexyl, heptyl, octyl, etc.

Examples of the alkenyl group of the above mentioned (2) include straight or branched lower alkenyl group having about 2-8 carbon atoms such as vinyl, propenyl, 2-butenyl, 3-butenyl, isobutenyl, 2-octenyl, etc.

Examples of the alkynyl group of the above mentioned (3) include straight or branched lower alkynyl group having about 2-8 carbon atoms such as ethynyl, 2-propynyl, 2-butynyl, 2-pantynyl, 2-octynyl, etc.

Examples of the cycloalkyl group of the above (4) include a lower cycloalkyl having about 3-6 carbon atoms, such as cyclopropyl, cyclobutyl, cyclopentyl, cyclohexyl, etc.

Each of the above-mentioned alkyl group, alkenyl group, alkynyl group and cycloalkyl group may be substituted with hydroxyl group, an optionally substituted amino group (e.g., amino, N-lower (C₁₋₄) alkylamino, N,N-di-lower (C₁₋₄) alkylamino, etc.), halogen , lower (C₁₋₄) alkoxy group, lower (C₁₋₄) alkylthio group, etc.

Examples of the aralkyl group of the above (5) include a phenyl-lower (C₁₋₄) alkyl, etc., such as benzyl, phenethyl, etc.

Examples of the aryl group of the above (6) include phenyl, etc.

Each of the above-mentioned aralkyl group and aryl group may be substituted, at any possible position on the benzene ring, with a halogen (e.g., F, Cl, Br, etc.), a nitro, an optionally substituted amino group (e.g., amino, N-lower (C₁₋₄) alkylamino, N,N-di-lower (C₁₋₄) alkylamino, etc.), a lower (C₁₋₄) alkoxy (e.g., methoxy, ethoxy, etc.), a lower (C₁₋₄) alkylthio (e.g., methylthio, ethylthio, etc.), a lower (C₁₋₄) alkyl (e.g., methyl, ethyl, etc.), etc.

Preferred examples of the "optionally substituted hydrocarbon residue" in the "optionally substituted hydrocarbon residue which may bind through a hetero-atom" represented by R³ include an optionally substituted alkyl or alkenyl group (e.g., a lower (C₁₋₅) alkyl or a lower (C₂₋-₅) alkenyl group, each of which may be substituted with a hydroxyl group, an amino group, a halogen, a lower (C₁₋₄) alkoxy group, etc.). Among others, a lower (C₁₋₅) alkyl (more preferably, ethyl) is preferable.

Preferred examples of the "hetero-atom" in the "optionally substituted hydrocarbon residue which may bind through a hetero-atom" represented by R³ include -O-, -S(O)m- [m is an integer of 0 to 2], -NR'- [R' is a hydrogen atom or a lower (C₁₋₄) alkyl], etc. Among others, -O- is preferable.

Among others, as R³, -a lower (C₁₋₅) alkyl or a lower (C₂₋₅) alkenyl group, each of which may be substituted with a substituent selected from the class consisting of a hydroxyl group, an amino group, a halogen and a lower (C₁₋₄) alkoxy group and which may bind through -O-, -S(O)ₘ- [m is an integer of 0-2] or -NR'- [R' is a hydrogen atom or a lower (C₁₋₄) alkyl], etc. is preferable and a lower (C₁₋₅) alkyl or lower (C₁₋₅) alkoxy (in particular, ethoxy) is more preferable.

Among the compounds having angiotensin II antagonistic activity and represented by the formula (I), a benzimidazole-7-carboxylic acid derivative of the formula (I'): 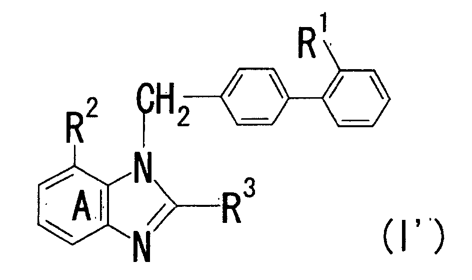 wherein R¹ is (1) carboxyl group, (2) tetrazolyl group or (3) a group of the formula: 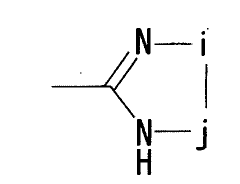 wherein i is -O- or -S-, j is >=O, >=S or >=S(O)m, and m is as defined above; the ring A is a benzene ring having an optional substituent selected from the class consisting of an optionally substituted lower (C₁₋₄) alkyl (e.g., a lower (C₁₋₄) alkyl optionally substituted with a hydroxyl group, a carboxyl group, a halogen, etc.) and a halogen, in addition to the group R² (preferably, a benzene ring having no substituent in addition to the group R²) ; R² is a group of the formula: -CO-D wherein D is (1) a hydroxyl group or (2) a lower (C₁₋₄) alkoxy whose alkyl moiety may be substituted with a hydroxyl group, an amino, a halogen, a lower (C₂₋₆) alkanoyloxy (e.g., acetoxy, pivaloyloxy, etc.), a lower (C₃₋₈) cycloalkanoyloxy, a lower (C₁₋₆) alkoxycarbonyloxy (e.g., methoxycarbonyloxy, ethoxy-carbonyloxy, etc.), a lower (C₃₋₈) cycloalkoxycarbonyloxy (e.g., cyclohexyloxycarbonyloxy, etc.), a lower (C₁₋₄) alkoxy or a lower (C₃₋₈) cycloalkoxy; R³ is a lower (C₁₋₅) alkyl or a lower (C₂₋₅) alkenyl group, each of which may bind through -O-, -S(O)m- [m is an integer of 0-2] or -NR'- [R' is a hydrogen atom or a lower (C₁₋₄) alkyl] and which may be substituted with a substituent selected from the class consisting of a hydroxyl group, an amino group, a halogen and a lower (C₁₋₄) alkoxy group (preferably, a lower (C₁₋₅) alkyl or a lower (C₁₋₅) alkoxy; more preferably, ethoxy), etc. or a pharmaceutically acceptable salt thereof is preferable.

Among others, 2-ethoxy-1-[[2'-(1H-tetrazol-5-yl)biphenyl-4-yl]methyl]benzimidazole-7-carboxylic acid [Candesartan], 1-(cyclohexyloxycarbonyloxy)ethyl 2-ethoxy-1-[[2'-(1H-tetrazol-5-yl)biphenyl-4-yl]methyl]benzimidazole-7-carboxylate [Candesartan cilexetil], pivaloyloxymethyl 2-ethoxy-1-[[2'-(1H-tetrazol-5-yl)biphenyl-4-yl]methyl]benzimidazole-7-carboxylate, 2-ethoxy-1-[[2'-(2,5-dihydro-5-oxo-1,2,4-oxadiazol-3-yl)biphenyl-4-yl]methyl]benzimidazole-7-carboxylic acid, etc. or a salt thereof are preferable.

The above mentioned benzimidazole derivative can be produced by known methods described in, for example, EP-425921, EP-459136, EP-553879, EP-578125, EP-520423, EP-668272, etc. or a method analogous thereto. When Candesartan cilexetil is used for the present invention, a stable C-type crystal described in EP-459136 is preferably used.

The compound having angiotensin II antagonistic activity or a pro-drug thereof may be distinct entity or in the form of any possible pharmaceutically acceptable salts thereof. Examples of said salts include a salt with inorganic bases (e.g., alkaline metals such as sodium, potassium, etc.; alkaline earth metals such as calcium, magnesium, etc.; transition metal such as zinc, iron, copper, etc.; etc.); organic bases (e.g., organic amines such as trimethylamine, triethylamine, pyridine, picoline, ethanolamine, diethanolamine, triethanolamine, dicyclohexylamine, N,N'-dibenzylethylenediamine, etc.; basic amino acids such as arginine, lysine, ornithine, etc.; etc.); etc., when said compound having angiotensin II antagonistic activity has an acidic group such as a carboxyl group, etc.; and a salt with inorganic acids or organic acids (e.g., hydrochloric acid, nitric acid, sulfuric acid, phosphoric acid, carbonic acid, bicarbonic acid, formic acid, acetic acid, propionic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid, etc.); acidic amino acids such as aspartic acid, glutamic acid, etc.; etc., when said compound having angiotensin II antagonistic activity has a basic group such as an amino group, etc.

The pro-drug of the compound having angiotensin II antagonistic activity [hereinafter, referred to as All antagonist] means a compound which is converted to All antagonist under the physiological condition or with a reaction due to an enzyme, an gastric acid, etc. in the living body, that is, a compound which is converted to All antagonist with oxidation, reduction, hydrolysis, etc. according to an enzyme; a compound which is converted to All antagonist with gastric acid, etc.; etc.

Examples of the pro-drug of the All antagonist include a compound wherein an amino group of the All antagonist is substituted with acyl, alkyl, phosphoric acid, etc. (e.g. a compound wherein an amino group of the All antagonist is substituted with eicosanoyl, alanyl, pentylaminocarbonyl, (5-methyl-2-oxo-1,3-dioxolen-4-yl)methoxycarbonyl, tetrahydrofuranyl, pyrrolidylmethyl, pivaloyloxymethyl, tert-butyl, etc.); a compound wherein an hydroxyl group of the All antagonist is substituted with acyl, alkyl, phosphoric: acid, boric acid, etc. (e.g. a compound wherein hydroxyl group of the All antagonist is substituted with acetyl, palmitoyl, propanoyl, pivaloyl, succinyl, fumaryl, alanyl, dimethylaminomethylcarbonyl, etc.); a compound wherein a carboxyl group of the All antagonist is modified with ester, amide, etc. (e.g. a compound wherein carboxyl group of the All antagonist is modified with ethyl ester, phenyl ester, carboxymethyl ester, dimethylaminomethyl ester, pivaloyloxymethyl ester, ethoxycarbonyloxyethyl ester, phthalidyl ester, (5-methyl-2-oxo-1,3-dioxolen-4-yl)methyl ester, cyclohexyloxycarbonylethyl ester, methyl amide, etc.); etc. These pro-drug can be produced by per se known method from the All antagonist.

The pro-drug of the All antagonist may be a compound which is converted into the AII antagonist under the physiological conditions as described in "Pharmaceutical Research and Development", Vol. 7 (Drug Design), pages 163-198 published in 1990 by Hirokawa Publishing Co. (Tokyo, Japan).

Also, the All antagonist may be hydrated.

The compound represented by the formula (I) or a pharmaceutically acceptable salt thereof is low in toxicity and can be administered as it is, or as a pharmaceutical composition thereof with a pharmaceutically acceptable carrier, to mammals (e.g., men, mice, rats, rabbits, dogs, cats, bovines, pigs, monkeys, etc.) as a fibrinogen lowering agent.

Here, examples of the pharmaceutically acceptable carrier include various organic or inorganic carriers which are generally used in this field. For example, an excipient, a lubricant, a binder, an disintegrating agent, etc. are used in solid formulations, and a solvent, a solubilizer, a suspending agent, a isotonizing agent, a buffer, a soothing agent, etc. are used in liquid formulations. In addition, if desired, an appropriate additive such as a preservative, an antioxidant, a colorant, a sweetener, etc. may be used in the above formulations.

Examples of the excipient include lactose, sucrose, D-mannitol, D-sorbitol, starch, α-starch, dextrin, crystalline cellulose, hydroxypropyl cellulose with a low degree of substitution, sodium carboxymethyl cellulose, gum arabic, dextrin, pullulan, light silic acid anhydride, synthesized aluminum silicate, magnesium aluminate metasilicate, etc.

Examples of the lubricant include magnesium stearate, calcium stearate, talc, colloidal silica, etc.

Examples of the binder include α-starch, sucrose, gelatin, gum arabic, methyl cellulose, carboxymethyl cellulose, sodium carboxymethyl cellulose, crystalline cellulose, sugar, D-mannitol, trehalose, dextrin, pullulan, hydroxypropyl cellulose, hydroxypropylmethyl cellulose, polyvinyl--pyrrolidone, etc.

Examples of the disintegrating agent include lactose, sugar, starch, carboxymethyl cellulose, carboxymethyl cellulose calcium, croscarmellose sodium, sodium carboxymethyl starch, light silic acid anhydride with a low degree of substitution, hydroxypropyl cellulose, etc.

Examples of the solvent include water for injection, Ringer solution, alcohol, propylene glycol, polyethylene glycol, sesame oil, corn oil, olive oil, cottonseed oil, etc.

Examples of the solubilizer include polyethylene glycol, propylene glycol, D-mannitol, trehalose, benzyl benzoate, ethanol, trisaminomethane, cholesterol, triethanolamine, sodium carbonate, sodium citrate, sodium salicylate, sodium acetate, etc.

Examples of the suspending agent include surfactants such as stearyl triethanolamine, sodium laurylsulfate, laurylaminopropionic acid, lecithin, benzalkonium chloride, benzetonium chloride, glycerin monostearate, etc.; hydrophilic polymers such as polyvinylalcohol, polyvinylpyrrolidone, sodium carboxymethyl cellulose, methyl cellulose, hydroxymethyl cellulose, hydroxyethyl cellulose, hydroxypropyl cellulose, etc.; polysorbates, polyoxyethylene hardened caster oil, etc.; etc.

Examples of the isotonizing agent include sodium chloride, glycerin, D-mannitol, D-sorbitol, glucose, etc.

Examples of the buffer include a buffer solution of phosphate, acetate, carbonate, citrate, etc.

Examples of the soothing agent include benzylalcohol, etc.

Examples of the preservative include paraoxybenzoic acid esters, chlorobutanol, benzylalcohol, phenethylalcohol, dehydroacetic acid, sorbic acid, etc.

Examples of the antioxidant include sulfites, ascorbic acid, etc.

Preferable examples of colorants include water-soluble synthetic organic food additives (e.g., food dyes such as food red dye Nos. 2 and 3, food yellow dye Nos. 4 and 5 and food 'blue dye Nos. 1 and 2), water-insoluble lake dyes (e.g., aluminum salts of the above water-soluble synthetic organic food additives, etc.), natural pigments (e.g., β-carotene, chlorophyll, iron oxide red, etc.), etc.

Preferable examples of sweeteners include sodium saccharate, glycyrrhizin dipotassium, aspartame, Stevia, etc.

The pharmaceutical composition is orally or parenterally administered in safety in the form of, for example, orally administered compositions such as tablets, capsules (including soft capsules and microcapsules), granules, powders, syrups, emulsions, suspensions, etc.; and parenterally administered compositions such as injectable preparations (e.g., sustained release injectable preparation, subcutaneous injectable preparation, intravenous injectable preparation, intramuscular injectable preparation, intraperitoneal injectable preparation, intravitreous injectable preparation, etc.), drops, medicines for external use (e.g., compositions for nasotracheally administration, compositions for percutaneously administration, ointments, etc.), suppositories (e.g., rectal suppository, vaginal suppository, etc.), pellets, drops, and the like.

The pharmaceutical composition can be prepared according to any of conventional methods in the field of pharmaceutical compositions, for example, according to the procedure described in the Japanese Pharmacopoeia. Hereinafter, a specific method of preparing the pharmaceutical composition will be described in detail.

The pharmaceutical composition contains the compound represented by the formula (I) or a pharmaceutically acceptable salt thereof in an amount of about 0.001% by weight to about 95% by weight, preferably about 0.1% by weight to about 70% by weight based on the total weight of the composition.

For example, a pharmaceutical composition to be orally administered is prepared by adding to the active component an excipient (e.g., lactose, sugar, starch, D-mannitol, etc.), disintegrating agent (e.g., carboxymethyl cellulose calcium, etc.), binder (e.g., α-starch, gum arabic, carboxymethyl cellulose, hydroxypropyl cellulose, polyvinyl-pyrrolidone, etc.), lubricant (e.g., talc, magnesium stearate, polyethylene glycol 6000, etc.), etc., and compression-molding the mixture composition, and if necessary, coating the composition with a coating base material by a known method so as to mask the taste, to form enteric coating, or to obtain long lasting activity.

Examples of the coating base material include sugar coating material, water-soluble film coating material, enteric film coating material, sustained release film coating material, etc.

As the sugar coating material, sugar is used, which may be used in combination with at least one material selected from talc, precipitated calcium carbonate, gelatin, gum arabic, pullulan, carnauba wax, etc.

Examples of the water-soluble film coating material include cellulose polymers such as hydroxypropyl cellulose, hydroxypropylmethyl cellulose, hydroxyethyl cellulose, and methyl hydroxyethyl cellulose; synthesized polymers such as polyvinyl acetal diethylaminoacetate, aminoalkylmethacrylate copolymer E [Eudragit E (trade name), Rhom Pharma], polyvinyl-pyrrolidone, etc.; polysaccharides such as pullulan, etc.

Examples of the enteric film coating material include cellulose polymers such as hydroxypropylmethyl cellulose phthalate, hydroxypropylmethyl cellulose acetate succinate, carboxymethylethyl cellulose, cellulose acetate phthalate, etc.; acrylic polymers such as methacrylate copolymer LD [Eudragit L-30D55 (trade name), Rhom Pharma], methacrylic copolymer S [Eudragit S (trade name), Rhom Pharma]; and natural substances such as shellac, etc.

Examples of the sustained release film coating materials include cellulose polymers such as ethyl cellulose; and acrylate polymers such as aminoalkyl methacrylate copolymer RS[Eudragit RS (trade name), Rhom Pharma], ethyl acrylate methyl methacrylate copolymer suspension [Eudragit NE (trade name), Rhom Pharma], etc.

Each of the above coating materials may be used as a mixture with at least two thereof in a proper ratio. In addition, a light-shielding material such as titanium oxide, iron sesquioxide or the like may be used in the course of coating.

The injectable preparation is prepared by dissolving, suspending or emulsifying the active component in an aqueous solvent (e.g., distilled water, physiological salt solution, Ringer solution, etc.), an oil solvent (e.g., vegetable oils such as olive oil, sesame oil, cottonseed oil, corn oil, and propylene glycol, etc.) or the like together with a dispersant (e.g., polysorbate 80, polyoxyethylene hardened caster oil 60, etc.), polyethylene glycol, carboxymethyl cellulose, sodium alginate, etc.), preservative (e.g., methyl paraben, propyl paraben, benzylalcohol, chlorobutanol, phenol, etc.), isotonizing agent (e.g., sodium chloride, glycerin, D-mannitol, D-sorbitol, glucose, etc.) or the like. In this preparation, if necessary, additives such as a solubilizer (e.g., sodium salycylate, sodium acetate, etc.), stabilizer (e.g., human serum albumin, etc.), soothing agent (e.g., benzylalcohol, etc.) and the like may be used.

The pharmaceutical composition of the present invention is preferably formulated into a sustained release preparation.

Examples of the sustained release preparation include:
[1] a sustained release preparation comprising a compound represented by the formula (I) or a salt thereof and a biodegradable polymer;
[2] the sustained release preparation according to item [1], wherein the biodegradable polymer is an α-hydroxycarboxylic acid polymer;
[3] the sustained release preparation according to item [1], wherein the α-hydroxycarboxylic acid polymer is a lactic acid-glycolic acid polymer;
[4] the sustained release preparation according to item [3], wherein the molar ratio of the lactic acid to the glycolic acid is from 100/0 to 40/60;
[5] the sustained release preparation according to item [2], wherein the polymer has a weight average molecular weight of 3,000 to 50,000;
[6] the sustained release preparation according to item [1], wherein the preparation is for injectable use;
[7] the sustained release preparation according to item [1], wherein the preparation contains a multivalent metal;
[8] the sustained release preparation according to item [7], wherein the multivalent metal is zinc; and
[9] a sustained release preparation, comprising a compound represented by the formula (I) or a salt thereof, a biodegradable polymer, and a multivalent metal.

These sustained release preparations can be prepared and used according to the description in EP-A-1058541.

Examples of another aspect of the sustained release preparation include:
[1] a sustained release preparation comprising a compound represented by the formula (I) or a salt thereof, a component obtained by treating a slightly water-soluble multivalent metal compound with water, and a biodegradable polymer;
[2] the sustained release preparation according to item [1], wherein the biodegradable polymer is an α-hydroxycarboxylic acid polymer;
[3] the sustained release preparation according to item [2], wherein the α-hydroxycarboxylic acid polymer is a lactic acid-glycolic acid polymer;
[4] the sustained release preparation according to item [3], wherein the mola.r ratio of the lactic acid to the glycolic acid,is from 100/0 to 40/60;
[5] the sustained release preparation according to item [2], wherein the polymer has a weight average molecular weight of 3,000 to 50,000;
[6] the sustained release preparation according to item [1], wherein the preparation is for injectable use;
[7] the sustained release preparation according to item [1], wherein the multivalent metal is zinc;
[8] the sustained release preparation according to item [1], wherein the multivalent metal compound is zinc oxide;
[9] the sustained release preparation according to item [1], further including a multivalent metal; and
[10] the sustained release preparation according to item [9], wherein the multivalent metal is zinc.

These sustained release preparations can be prepared and used according to the description in PCT/JP01/01191.

The dosage of the compound represented by the formula (I) or a pharmaceutically acceptable salt thereof varies depending on a particular subject, administration route, disease, symptom, etc. For example, the active component of the compound represented by the formula (I) or a pharmaceutically acceptable salt thereof is orally administered to a mammal, specifically an adult (body weight 50 kg) with hyperfibrinogenemia in a dose of about 0.001 to 500 mg, preferably 0.1 to 50 mg, preferably once to three times per day.

The fibrinogen-lowering agent of the present invention is used as a prophylactic or therapeutic agent for fibrinogen-related diseases of mammals (e.g. human, mouse, rat, rabbit, dog, cat, bovine, horse, pig, monkey, etc.). The fibrinogen-related diseases include any disease that is induced in the presence of fibrinogen and can be treated through the effect of lowering fibrinogen level. Examples of such a disease include hypercardia, chronic heart failure including stagnant heart failure, angina pectoris, arrhythmia, myocardial infarction, hyperfibrinogenemia induced ischemic heart diseases of nephrosis patients, silent cerebrovascular accident, transient brain ischemic attack, RIND, stroke, brain vascular dementia, neural symptoms in acute phase of brain thrombosis, lacunar infarct, brady-progressive brain thrombosis, hyperfibrinogenemia induced ischemic brain and heart diseases, cerebral edema, brain circulatory disorders, recurrence and sequelae of cerebrovascular disorders (e.g. neural symptoms, mental symptoms, subjective symptoms, and disorders in daily life activities), ischemic peripheral circulatory disorders, myocardial ischemia, venous dysfunction, progress of heart failure after myocardial infarction, diabetes, diabetic complications (e.g. diabetic retinopathy, diabetic nephropathy, and diabetic neural disorders), nephritis, glomerulonephritis, glomerulosclerosis, renal failure, microangiopathy, dialysis complications, hyperfibrinogenemia induced ischemic brain and heart diseases of dialysis patients, hyperfibrinogenemia induced deterioration of nephrosis patients' kidney function, radiation induced organopathy including nephropathy, acute renal failure associated with endotoxin shock, acute renal failure associated with ischemia, deterioration of renal diseases caused by fibrin deposition on renal glomerulus, prophylaxis of blood coagulation in using apparatus for hemodialysis or other extracorporeal circulations, prophylaxis of blood coagulation in transfusion or blood tests, limb artery thromboembolism, peri- or post-operative thromboembolism, prophylaxis of blood coagulation in intervascular catheterization, arteriosclerosis including atherosclerosis, aneurysm, coronary arteriosclerosis, brain arteriosclerosis, sudden deafness, pulmonary or brain embolism caused by cardiogenic shock or atrial fibrillation, profunda vein thrombosis, brain or pulmonary embolism associated with profunda vein thrombosis, pulmonary embolism, intermittent claudication, vibration disease, bedsore, phlebothrombosis, arterial thrombosis, generalized intravascular coagulation syndrome, phlebothrombosis in replacement arthroplasty, prophylaxes of thrombi- or fibrin-dependent metastases of various cancers, vascular hypertrophy, vascular hypertrophy and thrombus induced vascular obstruction and organopathy after intervention (e.g. percutaneous transluminal coronary angioplasty, percutaneous transluminal coronary revascularization, stent implantation, coronary endoscopy, intravascular ultrasonography, and coronary injection thrombolytic therapy), vascular reobstruction or reconstriction after bypass surgery, organopathy or vascular hypertrophy after grafting, thrombosis, multiorgan failure, endothelium dysfunction, obstructive peripheral circulatory disorder, ischemic brain circulatory disorders, limb ulcer associated with chronic artery obstruction (e.g. Buerger's disease and arteriosclerosis obliterans), resting gout and psychroesthesia, lower limb gangrene, bloodstream disorders .caused by cerebrovascular contraction after subarachnoid hemorrhage surgery, metabolic disorders (e.g. obesity, syndrome X, hyperlipemia, hypercholesterolemia, diabetes, and glucose tolerance abnormality), central nervous disorders (e.g. such disorders as brain infarct and sequelae and complications thereof, head injury, spinal injury, cerebral edema, sensory dysfunction, autonomic dysfunction, and multiple sclerosis), dementia, memory disorder, consciousness disorder, amnesia, anxiety symptoms, catatonic symptoms, dysphoric mental symptoms, acute inflammatory reactions, retinopathy, nephropathy, neuropathy, diabetes complications such as great vessel disorders, chronic articular rheumatism, allergic rhinitis, anaphylaxis, collagenosis, renal failure associated with systemic lupus erythematosus, hepatitis including chronic hepatitis, cirrhosis, Kawasaki disease, thrombosis in pregnancy, thrombosis in oral contraceptive intake, septicemia, acute renal failure associated with sepsis, endotoxin shock, Gram-negative sepsis, toxin shock syndrome, Ménière syndrome, vertigo, balance disorders, dysphagia, deafness, keloid, angioma, and the like; and hyperfibrinogenemia inducible cardiovascular diseases, cerebrovascular diseases, kidney or urinary diseases, arteriosclerotic diseases, eye diseases, metabolic or nutritional disorders, neural degeneration diseases, central nervous disorders, endocrinopathy, inflammatory diseases, allergic diseases, liver diseases, alimentary organ diseases, blood or hematinic organ diseases, bone diseases, tumor, gynecologic diseases, virilia diseases, respiratory organ diseases, environmental or occupational factor induced diseases, infectious diseases, toxemia, otolaryngological diseases, skin diseases, and the like.

The present invention also includes a prophylactic or therapeutic agent for hyperfibrinogenemia accompanied by hypercholesterolemia or hyperfibrinogenemia accompanied by a renal disorder (e.g. a renal dysfunction associated with glomerulonephritis, glomerulosclerosis, interstitial nephritis, nephrosclerosis, or polycystic renal diseases), which comprises a compound having renin-angiotensin inhibitory activity, a prodrug thereof, or a salt thereof.

Examples of the compound having renin-angiotensin inhibitory activity to be used include one or more (preferably two or three) compounds selected from the group consisting of (1) the above-mentioned All antagonist compound, (2) a compound having angiotensin converting enzyme (ACE) inhibitory activity, (3) a compound having renin inhibitory activity, (4) a compound having chymase inhibitory activity, and (5) compound having an aldosterone antagonistic activity.

Examples of the ACE-inhibiting compound include enalapril, lisinopril, and omapatrilat (BMS-186716), whose albuminuria suppressing activities have been described in many reports, and compounds (protease inhibitors) having both ACE inhibitory activity and NEP (neutral endopeptidase) inhibitory activity. The compound having both ACE and NEP inhibitory activities is known as a drug for treating hypertension by inhibiting production of angiotensin II and decomposition of atrial natriuretic peptide (ANP having the effects of reducing humor volume and vasodilation) and expected for its utility to therapeutic pharmaceuticals for failures, for example, including omapatrilat and sampatrilat (UK-81252; Pfiser).

Examples of the compound having renin inhibitory activity include SPP-100 (Novartis), etc.

Examples of the compound having chymase inhibitory activity include NK3201 (NIPPON KAYAKU), etc.

Examples of the compound having aldosterone antagonistic activity include SC-66100 (Pharmacia), etc.

Prodrugs or salts of these compounds may also be used and examples thereof include those exemplified with respect to the above-mentioned compound having All antagonistic activity.

The compound having renin-angiotensin inhibitory activity, a prodrug thereof, or a salt thereof is less toxic and can be used for a prophylactic or therapeutic agent for hyperfibrinogenemia accompanied by hypercholesterolemia or hyperfibrinogenemia accompanied by a renal disorder in mammals (e.g. human, mouse, rat, rabbit, dog, cat, bovine, pig, monkey, etc.) as it is or in the form of a pharmaceutical composition comprising it and a pharmaceutically acceptable carrier.

"The prophylactic or therapeutic agent for hyperfibrinogenemia accompanied by hypercholesterolemia or hyperfibrinogenemia accompanied by a renal disorder" encompasses "a prophylactic or therapeutic agent for hyperfibrinogenemia accompanied by hypercholesterolemia", "a prophylactic or therapeutic agent for hyperfibrinogenemia accompanied by a renal disorder", and a prophylactic or therapeutic agent for both diseases of "hyperfibrinogenemia accompanied by hypercholesterolemia" and "hyperfibrinogenemia accompanied by a renal disorder".

Examples of the pharmaceutically acceptable carrier include those exemplified with respect to the above-mentioned "fibrinogen lowering agent comprising the compound having All antagonistic activity, a prodrug thereof, or a salt thereof".

Examples of the dosage form of the pharmaceutical composition include oral dosage forms such as tablets, capsules (including soft capsules and microcapsules), granules, powders, syrups, emulsions, suspensions, etc.; and parenteral forms such as injectable preparations (e.g. sustained release injectable preparations, subcutaneous injectable preparations, intravenous injectable preparations, intramuscular injectable preparations, intraperitoneal injectable preparations, intravitreous injectable preparations, etc.), drops, external preparations (e.g. compositions for nasal administration, compositions for percutaneous administration, ointments, etc.), suppositories (e.g. rectal suppositories, vaginal suppositories, etc.), pellets, drops, etc. The pharmaceutical composition can safely be orally or parenterally administered in each of these dosage forms.

The pharmaceutical composition contains the compound having renin-angiotensin inhibitory activity, a prodrug thereof, or a salt thereof in an amount of about 0.001% by weight to about 95% by weight, preferably about 0.1 by weight to about 70% by weight based on the total amount of the composition.

The pharmaceutical composition can be prepared by conventional methods in the manufacturing technique field such as the method according to Japanese Pharmacopoeia. The above-mentioned preparations can be prepared by the same method as that exemplified with respect to the above "fibrinogen lowering agent comprising the compound having All antagonistic activity, a prodrug thereof, or a salt thereof".

The dosage of the compound having renin-angiotensin inhibitory activity varies depending on a particular subject, administration route, disease, symptom, etc. For example, the active component of the compound having renin-angiotensin inhibitory activity, or a prodrug thereof, or a salt thereof is orally administered to a mammal, specifically an adult (body weight 50 kg) with hyperfibrinogenemia accompanied by hypercholesterolemia or hyperfibrinogenemia accompanied by a renal disorder in a dose of about 0.001 to 500 mg, preferably 0.1 to 50 mg, preferably once to three times per day.

The compound having renin-angiotensin inhibitory activity can suppress angiotensin II renal hemodynamics-induced renal disorders such as hyperlucent based on the disorder of size selecting function (i.e., permeability to such macromolecules as proteins) of glomerular basal lamina and induce an albuminuria reducing effect to serve as a good prophylactic or therapeutic agent for hyperfibrinogenemia.

Further, the compound having renin-angiotensin inhibitory activity, which can reduce albuminuria, can also serve as a good prophylactic or therapeutic agent for hyperfibrinogenemia accompanied by hypercholesterolemia, which would otherwise be caused by an albuminuria-induced decrease in plasma proteins and acceleration of synthesis of cholesterols and plasma proteins in liver.

The fibrinogen lowering agent or the prophylactic or therapeutic agent for hyperfibrinogenemia accompanied by hypercholesterolemia or hyperfibrinogenemia accompanied by a renal disorder of the present invention may also be used in combination with any antithrombotic or fibrinolytic agent.

In addition other agents which can be used in combinat ion include lipid-lowering drugs, cholesterol-lowering drug s, HMG-CoA reductase (3-hydroxy-3-methylglutaryl coenzyme A reductase) inhibitors, insulin-sensitizing agents, therape utic agents for bone diseases, heart muscle protecting agen ts, therapeutic agents for coronary diseases, therapeutic a gents for hypertension, therapeutic agents for chronic hear t failures, therapeutic agents for diabetes, therapeutic ag ents for hypothyroidism, therapeutic agents for nephrotic s yndrome, therapeutic agents for chronic renal failures, the rapeutic agents for gynecologic diseases, and therapeutic a gents for infectious diseases. These agents may each be ad ministered in the form of an oral preparation or a suppository such as a rectal preparation as desired. In this case, examples of a component which can be used in combination include fibrates (e.g. clofibrate, bezafibrate, gemfibrozil, etc.), nicotinic acids and their derivatives and analogs (e.g. acipimox, probucol, etc.), bile acid coupling resins (e.g. colestyramine, colestipol, etc.), cholesterol absorpt ion suppressing compounds (e.g. sitosterol, neomycin, et c.), and squalene epoxidase inhibitors (e.g. NB-598 and der ivatives thereof, etc.).

Further, examples of other components which can be used in combination include oxidesqualene-lanosterol cyclases such as decalin derivatives, azadecalin derivatives, indan derivatives, etc.

Each of the following therapeutic agents may also be used in combination:
Prophylactic or therapeutic agents for thrombus formation: anticoagulants [e.g. heparin sodium, heparin calcium, warfarin calcium (Warfarin) , blood coagulation factor Xa inhibitors, and coagulation-fibrinogenolysis system balance correcting agents], fibrinolytic agents (e.g. tPA and urokinase), and antiplatelet agents [e.g. aspirin, sulphin-pyrazone (Anturan), dipyridamole (Persantin), ticlopidine (Panaldine), cilostazol (Pletaal), and GP IIb/IIIa antagonists (ReoPro)], etc.;
Brain circulatory or metabolism ameliorating agents: meclofenoxate, trapidil, pentoxifylline, etc.;
Therapeutic agents for intracranial edema: glycerin (Glypose), etc.;
Therapeutic agents for peripheral artery obstruction: argatroban (Slonnon), cilostazol (Pletaal), prostaglandin preparations (Prostandin), 5-HT2 antagonists (Sarpogrel), etc.;
Therapeutic agents for hypertension: diuretics [e.g. furosemide (Lasix), bumetanide (Lunetoron), and azosemide (Diart)], antihypertensive agents [e.g. ACE inhibitors (e.g. enalapril maleate (Renivace), etc.), Ca antagonists (e.g. manidipine, amlodipine, etc.), α or β receptor blockers, etc.], etc.;
Therapeutic agents for chronic heart failure: inotropic agents [e.g. cardiac glycosides (e.g. digoxin, etc.), β receptor stimulants (catecholamine preparations such as denopamine, dobutamine, etc.), PDE inhibitors, etc.], diuretics [e.g. furosemide (Lasix) and spironolactone (Aldactone), etc.], ACE inhibitors [e.g. enalapril maleate (Renivace), etc.], Ca antagonists (e.g. amlodipine, etc.), β receptor blockers, etc.;
Antiarrhythmic agents: disopyramide, lidocaine, quinidine sulfate, flecainide acetate, mexiletine hydrochloride, amiodarone hydrochloride, β blockers, Ca antagonists;
therapeutic agents for bone diseases: calcium preparations (e.g. calcium carbonate), calcitonin preparations, activated vitamin D₃ preparations [e.g. alfacalcidol (e.g. Alfarol), calcitriol (Rocaltrol), etc.], sex hormones (e.g. estrogen, estradiol, etc.), hormone preparations [e.g. conjugated estrogen (Premarin), etc.], ipriflavone preparations (e.g. Osten, etc.), vitamin K₂, vitamin K₂ preparations [e.g. menatetrenone (Glakay), etc.], bisphosphonic acid preparations (e.g. etidronates, etc.), prostaglandin A1, fluorides (e.g. sodium fluoride, etc.), osteogenesis proteins (BMP), fibroblast growth factor (FGF), platelet derived growth factor (PDGF), transforming growth factor (TGF-β), insulin-like growth factors-1 and -2 (IGF-1, -2), parathyroid hormone (PTH), the compounds disclosed in EP-A1-376197, EP-A1-460488, and EP-A1-719782 (e.g. (2R,4S)-(-)-N-[4-(diethoxyphosphorylmethyl)phenyl]-1,2,4,5-tetrahydro-4-methyl-7,8-methylenedioxy-5-oxo-3-benzothiepin-2-carboxamide, etc.), etc.;
Therapeutic agents for diabetes: Actos, Rosiglatazone, Kinedak, Penfill, Humulin, Euglucon, Glimicron, Daonil, Novoline, Monotard, insulins, Glucobay, Dimelin, Rastinon, Bacilcon, Deamelin S, Iszilin family, etc.;
Therapeutic agents for hypothyroidism: dried thyroid (Thyreoid), levothyroxine sodium (Thyradin-S), liothyronine sodium (Thyronine and Thyronamin), etc.;
Therapeutic agents for nephrotic syndrome: as agents for steroid therapy that is normally first-choice therapy, there are used prednisolone (Predonine), prednisolone sodium succinate (Predonine), methylprednisolone sodium succinate (Solu-Medrol), betamethasone (Rinderon), etc. For anticoagulant therapy, there are used antiplatelets and anticoagulants such as dipyridamole (Persantin), dilazep dihydrochloride (Comelian), Ticlopidine, Clopidogrel, FXa inhibitor, etc.;
HMG-CoA reductase inhibitors: cerivastatin, atorvastatin, pravastatin, simvastatin, itavastatin, lovastatin, fluvastatin, (+)-3R,5S-7-[4-(4-fluorophenyl)-6-isopropyl-2-(N-methyl-N-methanesulfonylamino)pyrimidine-5-yl]-3,5-dihydroxy-6(E)-heptenoic acid, etc.;
Therapeutic agents for chronic renal failure: diuretics [e.g. furosemide (Lasix), bumetanide (Lunetoron), azosemide (Diart)], antihypertensive agents [e.g. ACE inhibitors (e.g. enalapril maleate (Renivace))], Ca antagonists (e.g. manidipine), α receptor blockers, etc., f these agents are preferably be used by oral administration in case of combination use;
Coronary vessel dilators: nifedipine, diltiazem, nicorandil, nitrite agents, etc.;
Heart muscle protecting agents: opening agents for cardiac ATP-K, Na-H exchange inhibitors, endothelin antagonists, urotensin antagonists, etc.;
Anti-inflammatory agents: aspirin, acetaminophen, non-steroidal anti-inflammatory agents (e.g. indomethacin), steroidal agents (e.g. dexamethasone, etc.), etc.;
Antiallergic agents: antihistamines (e.g. chlorpheniramine maleate, etc.), stimulation therapy agents (e.g. bucillamine, etc.), azelastine hydrochloride, seratrodast, tranilast, oxatomide, Stronger Neo-Minophagen C, tranexamic acid, ketotifen fumarate, etc.;
Antineoplastic agents: alkylating agents, antimetabolites, antitumor antibiotics, antineoplastic plant component preparations, other antitumor agents, etc.;
Central nervous system agents: antianxiety agents, hypnotic-sedatives, narcotics, antispasmodics, automatic agents, antiparkinsonism agents, other agents for psychoneurosis, etc.;
Therapeutic agents for gynecologic diseases: therapeutic agents for climacteric disorders (e.g. estrogenes conjugated, estradiol, testosterone enanthate, estradiol valerate, etc.), therapeutic agents for breast cancer (e.g. tamoxifen citrate, etc.), therapeutic agents for endometriosis or hysteromyoma (e.g. leuprorelin acetate and danazol, etc.), etc.;
Therapeutic agents for infectious diseases: antibiotic preparations (e.g. cefotiam hydrochloride, cefozopran hydrochloride, ampicillin, etc.), chemotherapeutics (e.g. sulfa drugs, synthetic antimicrobial agents, antiviral agents, etc.), biological preparations (vaccines, blood preparations such as immunoglobulins, etc.), etc.; and
Further, there are anti-obesity drugs (e.g. mazindol, etc.); and antirheumatic agents; etc.

Each of these agents may be used at the same time or at appropriate time interval.

When these agents are used in combination, each of these agents can be used independently or mixed with each other to form a pharmaceutical composition by using a pharmaceutically acceptable carrier, vehicle, binder, diluent, or the like for oral or parenteral administration. When these agents are formulated into different preparations, separately prepared different preparations may be mixed with each other by using a diluent or the like upon administration. Alternatively, separately prepared different preparations may be administered to the same subject at the same time or at an appropriate time interval. The pharmaceutical composition of the present invention also include a kit product for administering separately prepared different preparations by mixing them with a diluent or the like upon administration (for example, an injectable kit comprising ampoules each containing a different powdered agent; and a diluent or the like for dissolving two or more powdered agents to form a mixture upon use); a kit product for administering separately prepared different preparations to the same subject at the same time or at an appropriate time interval (for example, a tablet kit for administrating two or more tablets at the same time or separately at an appropriate time interval which comprises two or more different tablets each containing a different agent, said tablets being contained in the same bag or different bags, if necessary, having a column for recording a given administration time(s)); and the like.

### Examples

The following examples and experimental examples further illustrate the present invention in detail but are not to be construed to limit the scope of the present invention.

The fibrinogen lowering agent or the prophylactic or therapeutic agent for hyperfibrinogenemia accompanied by hypercholesterolemia and/or hyperfibrinogenemia accompanied by a renal disorder comprising as an effective component the compound having renin-angiotensin system inhibitory activity (e.g. the compound having All antagonistic activity or a salt thereof) of the present invention can be produced, for example, according to the following formulations.

### Example 1

| Capsule | |
|---|---|
| (1) candesartan cilexetil | 30 mg |
| (2) lactose | 90 mg |
| (3) microcrystalline cellulose | 70 mg |
| (4) magnesium stearate | 10 mg |
| | 200 mg per capsule |

Components (1), (2) and (3), and 1/2 of component (4) were mixed and then granulated. To the granules was added the remainder of component (4), and the whole was filled into a gelatin capsule.

### Example 2

| Tablet | |
|---|---|
| (1) candesartan cilexetil | 30 mg |
| (2) lactose | 35 mg |
| (3) corn starch | 150 mg |
| (4) microcrystalline cellulose | 30 mg |
| (5) magnesium stearate | 5 mg |
| | 250 mg per capsule |

Components (1), (2), (3), and 2/3 of component (4), and 1/2 of component (5) were mixed and then granulated. To the granules were added the remainders of components (4) and (5), followed by subjecting the mixture to compression molding.

### Experimental Example 1

### Fibrinogen-Lowering Effect

### Method:

Spontaneously hypercholesterolemia (SHC) rats exhibit hypercholesterolemia and renal failure. Candesartan cilexetil (TCV-116; 0.5% methylcellulose 100 cp suspension; 1 mg/kg (2 ml/kg)) were administered orally to 10-week-old SHC rats once a day for a 6-week period. 0.5% methylcellulose 100 cp suspensions were administered to the control group (vehicle-treated group) in a volume of 2 ml/kg. Fibrinogen concentrations in plasma were measured as follows. At the end of experiments, the blood was withdrawn from aorta abdominalis into 3.8% trisodium citrate solution (Citral, Yamanouchi Pharmaceutical Co.,Ltd., final concentration of 0.38%) and the plasma were prepared by centrifuging at 3000 rpm at room temperature for 20 minutes. The fibrinogen concentration was determined using fibrinogen test RD (Roche Diagnostics) and the clot timer (B-10, Sarstedt, Inc.) with reference to a standard rat fibrinogen (F-6755, Sigma).

### Results:

The results are shown in Table 1.

**Table 1**

| Effect of TCV-116 on Fibrinogen Level of SHC Rat Plasma | | |
|---|---|---|
| | Control Group (Vehicle-treated) | TCV-116 1 mg/kg/day Administered Group |
| Fibrinogen level (mg/dL) | 496.4 ± 21.9 | 354.0 ± 29.1 ** |

| | | |
|---|---|---|
| **: p<0.01 vs vehicle by Student's t-test (Mean ± SE, n=5) | | |

As shown in Table 1, candesartan cilexetil (TCV-116, 1mg/kg) decreased the plasma fibrinogen level of SHC rat significantly in comparison with the control group (vehicle-treated group).

### Industrial Applicability

The fibrinogen lowering agent or the prophylactic or therapeutic agent for hyperfibrinogenemia accompanied by hypercholesterolemia or hyperfibrinogenemia accompanied by a renal failure of the present invention has excellent effect of lowering fibrinogen and is useful as a prophylactic or therapeutic agent for hyperfibrinogenemia accompanied by hypercholesterolemia or hyperfibrinogenemia accompanied by a renal failure, various diseases caused by hyperfibrinogenemia, and the like.

## Claims

1. A fibrinogen-lowering agent comprising a compound having angiotensin II antagonistic activity (exclusive of irbesartan), a prodrug thereof, or a salt thereof.

2. The agent according to claim 1, wherein the compound having angiotensin II antagonistic activity is a non-peptide compound.

3. The agent according to claim 1, wherein the compound having angiotensin II antagonistic activity is a compound having oxygen atom in its molecule.

4. The agent according to claim 1, wherein the compound having angiotensin II antagonistic activity is a compound having ether linkage or carbonyl group in its molecule.

5. The agent according to claim 1, wherein the compound having angiotensin II antagonistic activity is represented by the formula (I): 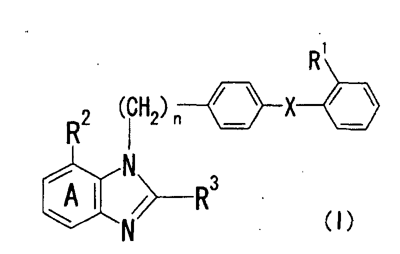 wherein R¹ is a group capable of forming an anion or a group convertible thereto, X shows that the phenylene group and the phenyl group are bonded directly or through a spacer having a chain length of 1 to 2 atoms, n is 1 or 2, ring A is benzene ring optionally further substituted, R² is a group capable of forming an anion or a group convertible thereto, and R³ is an optionally substituted hydrocarbon group which may be bonded through a hetero atom.

6. The agent according to claim 1, wherein the compound having angiotensin II antagonistic activity is losartan, eprosartan, candesartan cilexetil, candesartan, valsartan, telmisartan, olmesartan, or tasosartan.

7. The agent according to claim 1, wherein the compound having angiotensin II antagonistic activity is 2-etoxy-1-[[2'-(1H-tetrazol-5-yl)biphenyl-4-yl]methyl]benzimdazole-7-carboxylic acid.

8. The agent according to claim 1, wherein the compound having angiotensin II antagonistic activity is 1-(cyclohexyloxycarbonyloxy)ethyl 2-ethoxy-1-[[2'-(1H-tetrazol-5-yl)biphenyl-4-yl]methyl]benzimidazole-7-carboxylate.

9. The agent according to claim 1, wherein the compound having angiotensin II antagonistic activity is 2-ethoxy-1-[[2'-(2,5-dihydro-5-oxo-1,2,4-oxadiazol-3-yl)bipheny-4-yl]methyl]benzimidazole-7-carboxylic acid.

10. The agent according to claim 1, which is a prophylactic or therapeutic agent for hyperfibrinogenemia or a disease caused thereby.

11. A prophylactic or therapeutic agent for hyperfibrinogenemia accompanied by hypercholesterolemia, or hyperfibrinogenemia accompanied by a renal disorder, which comprises a compound having renin-angiotensin system inhibitory activity, a prodrug thereof, or a salt thereof.

12. The agent according to claim 11, wherein the compound having renin-angiotensin system inhibitory activity is one or more compounds selected from the group consisting of (1) a compound having angiotensin II antagonistic activity, (2) a compound having angiotensin converting enzyme inhibitory activity, (3) a compound having renin inhibitory activity, (4) a compound having chymase inhibitory activity, and (5) a compound having aldosterone antagonistic activity.

13. The agent according to claim 11, wherein the compound having renin-angiotensin system inhibitory activity is a compound having angiotensin II antagonistic activity.

14. The agent according to claim 13, wherein the compound having angiotensin II antagonistic activity is a non-peptide compound.

15. The agent according to claim 13, wherein the compound having angiotensin II antagonistic activity is a compound having oxygen atom in its molecule.

16. The agent according to claim 13, wherein the compound having angiotensin II antagonistic activity is a compound having ether linkage or carbonyl group in its molecule.

17. The agent according to claim 13, wherein the compound having angiotensin II antagonistic activity is represented by the formula (I): 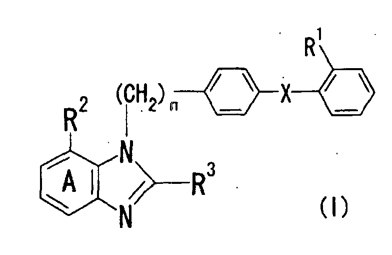 wherein R¹ is a group capable of forming an anion or a group convertible thereto, X shows that the phenylene group and the phenyl group are bonded directly or through a spacer having a chain length of 1 to 2 atoms, n is 1 or 2, ring A is benzene ring optionally further substituted, R² is a group capable of forming an anion or a group convertible thereto, and R³ is an optionally substituted hydrocarbon group which may be bonded through a hetero atom.

18. The agents according to claim 13, wherein the compound having angiotensin II antagonistic activity is losartan, eprosartan, candesartan cilexetil, candesartan, valsartan, telmisartan, irbesaltan, olmesartan, or tasosartan.

19. The agent according to claim 13, wherein the compound having angiotensin II antagonistic activity is 2-etoxy-1-[[2'-(1H-tetrazol-5-yl)biphenyl-4-yl]methyl]benzimdazole-7-carboxylic acid.

20. The agent according to claim 13, wherein the compound having angiotensin II antagonistic activity is 1-(cyclohexyloxycarbonyloxy)ethyl 2-ethoxy-1-[[2'-(1H-tetrazol-5-yl)biphenyl-4-yl]methyl]benzimidazole-7-carboxylate.

21. The agent according to claim 13, wherein the compound having angiotensin II antagonistic activity is 2-ethoxy-1-[[2'-(2,5-dihydro-5-oxo-1,2,4-oxadiazol-3-yl)bipheny-4-yl]methyl]benzimidazole-7-carboxylic acid.

22. The agent according to claim II, wherein the compound having renin-angiotensin system inhibitory activity is a compound having angiotensin converting enzyme inhibitory activity.

23. The agent according to claim 22, wherein the compound having angiotensin converting enzyme inhibitory activity is enalapril, lisinopril, omapatrilat, sampatrilat, or adecut.

24. The agent according to claim 22, wherein the compound having angiotensin converting enzyme inhibitory activity has both neutral endopeptidase inhibitory activity and angiotensin converting enzyme inhibitory activity.

25. The agent according to claim 24, wherein the compound having both angiotensin converting enzyme inhibitory activity and neutral endopeptidase inhibitory activity is omapatrilat or sampatrilat.

26. The agent according to claim 11, wherein the compound having renin-angiotensin system inhibitory activity is a compound having renin inhibitory activity.

27. The agent according to claim 26, wherein the compound having renin inhibitory activity is SPP-100.

28. The agent according to claim 11, wherein the compound having renin-angiotensin system inhibitory activity is a compound having chymase inhibitory activity.

29. The agent according to claim 28, wherein the compound having chymase inhibitory activity is NK3201.

30. The agent according to claim 11, wherein the compound having renin-angiotensin system inhibitory activity is a compound having aldosterone antagonistic activity.

31. The agent according to claim 30, wherein the compound having aldosterone antagonistic activity is SC-66100.

32. A method for preventing or treating hyperfibrinogenemia accompanied by hypercholesterolemia or hyperfibrinogenemia accompanied by a renal disorder, which comprises using a compound having renin-angiotensin system inhibitory activity, a prodrug thereof, or a salt thereof.

33. Use of a compound having renin-angiotensin system inhibitory activity, a prodrug thereof, or a salt thereof, for manufacturing a prophylactic or therapeutic agent for hyperfibrinogenemia accompanied by hypercholesterolemia or hyperfibrinogenemia accompanied by a renal disorder.

34. A method for lowering fibrinogen, which comprises using a compound having angiotensin II antagonistic activity (exclusive of irbesartan), a prodrug thereof, or a salt thereof.

35. Use of a compound having angiotensin II antagonistic activity (exclusive of irbesartan), a prodrug thereof, or a salt thereof, for manufacturing a fibrinogen-lowering agent.

36. A method for preventing or treating hyperfibrinogenemia or a disease caused thereby, which comprises administering an effective amount of a compound having angiotensin II antagonistic activity (exclusive of irbesartan), a prodrug thereof, or a salt thereof, to a mammal.

37. Use of a compound having angiotensin II antagonistic activity (exclusive of irbesartan), a prodrug thereof, or a salt thereof, for manufacturing a prophylactic or therapeutic agent for hyperfibrinogenemia or a disease caused thereby.
